# EUROPEAN PATENT SPECIFICATION

(11) **EP 4 213 773 B1**
(45) Date of publication and mention of the grant of the patent: **04.11.2026**
(21) Application number: 21787184.7
(22) Date of filing: 13.09.2021
(51) Int. Cl.: A61F 2/95, A61M 25/01, A61F 2/24, A61F 2/966

(54) **FLEXIBLE SHAFTS FOR MEDICAL DEVICE DELIVERY SYSTEMS**
FLEXIBLE WELLEN FÜR FREISETZUNGSSYSTEME FÜR MEDIZINISCHE VORRICHTUNGEN
ARBRES FLEXIBLES POUR SYSTÈMES DE POSE DE DISPOSITIF MÉDICAL

(30) Priority: 18.09.2020 US 202063080357 P
(43) Date of publication of application: 26.07.2023
(73) Proprietor: Medtronic Vascular Inc., Santa Rosa, CA 95403 (US)
(72) Inventor: SHALLY, Alice, Galway (IE); JENNINGS, Padraigh, Galway (IE); KING, Patrick, Galway (IE)
(74) Representative: Zimmermann & Partner Patentanwälte mbB
(86) International application number: PCT/US2021/049985
(87) International publication number: WO 2022/060645

(56) References cited:
- EP-A2- 2 663 259
- WO-A1-2019/147504
- US-A1- 2009 099 554
- US-A1- 2018 140 323

## Description

### FIELD

The present technology is generally related to medical devices. And, more particularly, to delivery systems for stents, prosthetic heart valves and other implantable medical devices.

### BACKGROUND

Patients suffering from various medical conditions or diseases may require surgery to install an implantable medical device. For example, valve regurgitation or stenotic calcification of leaflets of a heart valve may be treated with a prosthetic heart valve. A traditional surgical procedure to implant the prosthetic heart valve requires a sternotomy and a cardiopulmonary bypass, which creates significant patient trauma and discomfort. Traditional surgical procedures may also require extensive recuperation times and may result in life-threatening complications.

One alternative to a traditional surgical procedure is delivering implantable medical devices using minimally-invasive techniques. For example, a prosthetic heart valve can be percutaneously and transluminally delivered to an implant location. In such methods, the prosthetic heart valve can be compressed or crimped onto or into a delivery catheter of a delivery system for insertion within a patient's vasculature; advanced to the implant location; and re-expanded to be deployed at the implant location.

The delivery catheter of the delivery system typically includes a shaft that provide structural support to the delivery catheter. In some current devices, a shaft of a delivery system contains two spine wires running a length of both an inner and middle membrane that provides structural support. Due to rigidity and placement of the spine wires, the shaft can bend or flex in one plane only. This limited range motion may cause problems when tracking the implantable medical device through tortuous anatomies. For example, as the implantable medical device is tracked though anatomies which curve or bend in different planes, the delivery catheter may need to be rotated or repositioned in order to align the plane of motion of the shaft with the curve or bend of the anatomy. These additional maneuvers may increase both the duration of surgery and risk of vessel dissection.

US 2018/0140323 A1 describes a medical device shaft resistant to compression and/or tension.

WO 2019/147504 A1 describes cardiac valve delivery devices and systems.

### SUMMARY

The techniques of this disclosure generally relate to shafts for delivery systems that deliver an implantable medical device to an implant location.

In one aspect, the present disclosure provides a system for delivering an implantable medical device to an implant location. The system includes a control handle portion and a catheter portion coupled to the control handle portion at a proximal end of the catheter portion. The catheter portion includes an outer shaft. The outer shaft includes a plurality of segments arranged in an axial direction to form the outer shaft the catheter portion, each of the plurality of segments being configured to move relative to one another. The system also includes a distal portion coupled to a distal end of the outer shaft, the distal portion being configured to receive the implantable medical device.

In another aspect, the present disclosure provides a catheter for a delivery system for delivering an implantable medical device to an implant location. The catheter includes an outer shaft comprising a plurality of segments arranged in an axial direction from a control handle portion of the delivery system to a distal portion of the delivery system, each of the plurality of segments being configured to move relative to one another. The catheter also includes an inner shaft extending through an interior of the plurality of segments from the control handle portion to the distal portion.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings are not to scale.
FIGS. 1A-1C depict an illustration of a delivery system that may include outer shafts, according to an embodiment hereof.
FIG. 2 depicts an illustration of an outer shaft with segments for use with a delivery system, according to an embodiment hereof.
FIGS. 3A-3G depict several illustrations of segments for use with the outer shaft illustrated in FIG. 2, according to an embodiment hereof.
FIGS. 4A-4G depict several illustrations of other segments for use with the outer shaft illustrated in FIG. 2, according to an embodiment hereof.
FIGS. 5A-5E depict several illustrations of another outer shaft with segments for use with a delivery system, according to an embodiment hereof.
FIGS. 6A and 6B depict several illustrations of an implantable medical device that may be used with delivery systems, according to an embodiment hereof.

### DETAILED DESCRIPTION

Specific embodiments of the present disclosure are now described with reference to the figures. The following detailed description describes examples of embodiments and is not intended to limit the present technology or the application and uses of the present technology. Although the description of embodiments hereof is in the context of a delivery system that may be used with an implantable medical device, the present technology may also be used in other devices. Furthermore, there is no intention to be bound by any expressed or implied theory presented in the preceding technical field, background, brief summary or the following detailed description.

The terms "distal" and "proximal", when used in the following description to refer to a delivery system or catheter are with respect to a position or direction relative to the treating clinician. Thus, "distal" and "distally" refer to positions distant from, or in a direction away from the treating clinician, and the terms "proximal" and "proximally" refer to positions near, or in a direction toward the clinician.

Embodiments disclosed herein are directed to shafts for a delivery system for implantable medical devices. In embodiments, an outer shaft of a catheter portion of the delivery system includes repeating and interlocking segments that are positioned from a control handle portion to a distal portion of the delivery system. The repeating segments are configured to move independently relative to one another thereby allowing the catheter portion to bend or flex in multiple planes of motion relative to a central axis of the catheter portion. Due to the ability to bend or flex in multiple planes of motion, the delivery system enables a physician to steer the catheter portion without the need to re-assess and reposition the implantable medical device when tracking to an implant location. Because the operation of the delivery system may be a lengthy procedure, the ability to steer the catheter portion without repositioning the delivery system may increase the efficiency of the delivery system and may prove advantageous to the patient during recovery. Additionally, an increase in flexibility of the catheter portion may reduce the force required during tracking and steering thereby reducing the risk of vessel dissection.

In some embodiments, the repeating segments of the catheter portion are configured as interlocking ball and socket (or cup) joints, which allow flexibility in all directions. That is, each of the repeating segments includes a ball and a socket or cup. The ball of one repeating segment is configured to engage with a socket or cup of an adjacent repeating segment. The ball portion is configured to move within the socket or cup portion to allow adjacent repeating segments to move relative to one another. Replacing an outer shaft of the catheter portion with interlinking ball and socket/cup repeating segments can facilitate uniform and increased flexibility in the outer shaft which improves tracking and steering the catheter portion through tortuous anatomy.

In some embodiments, the repeating segments are configured as interlinking funnel segments. Each interlinking funnel segment is formed having an inner cavity that receives an end portion of an adjacent interlinking funnel segment. The interlinking funnel segments are coupled by an inner shaft passing through an interior of the interlinking funnel segments. As such, the interlinking segments form a non-continuous backbone, which can freely rotate around the inner shaft and can flex or bend in multiple planes of motion. As such, replacing an outer shaft of the catheter portion with interlinking funnel segments can facilitate uniform and increased flexibility in the outer shaft which improves tracking and steering the catheter portion through tortuous anatomy.

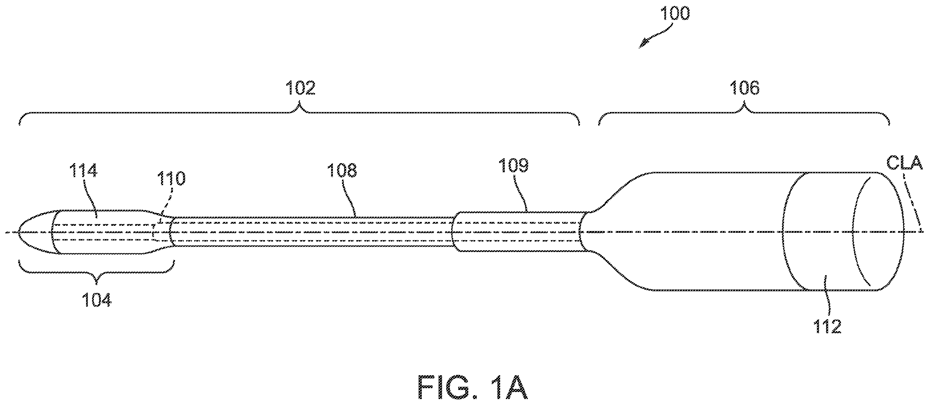

In embodiments described herein, the shafts are configured to operate in combination with a delivery system that operates to deliver an implantable medical device to an implant location. FIGS. 1A-1C illustrate an example of a delivery system 100 in accordance with an embodiment hereof. One skilled in the art will realize that FIGS. 1A-1C illustrate one example of a delivery system and that existing components illustrated in FIGS. 1A-1C may be removed and/or additional components may be added to the delivery system 100.

As shown in FIG. 1A, the delivery system 100 generally comprises a catheter portion 102 having a distal portion 104. The catheter portion 102 is coupled to a control handle portion 106 by which the catheter portion 102 is manipulated to deliver an implantable medical device (not shown), e.g., a prosthetic heart valve including a prosthetic valve structure and a stent, to an implant location and to deploy the implantable medical device at the implant location. In embodiments, as illustrated, the implantable medical device can be housed in a capsule 114.

The catheter portion 102 is preferably of a length and size so as to permit a controlled delivery of the distal portion 104 to the implant location, *e.g.,* a patient's heart. In embodiments, the catheter portion 102 includes an outer shaft 108 formed of interlinking segments to enhance maneuverability, steerability and advancement of the distal portion 104 to the implant location, as discussed below. The distal portion 104 provides the components by which an implantable medical device can be mounted for delivery to the implant location and further enables the expansion of the implantable medical device for effective deployment thereof. The control handle portion 106 preferably controls movement that is translated to the distal portion 104 by way of elongate structure of the catheter portion 102. Controlled functionality from the control handle portion 106 is preferably provided in order to permit expansion and deployment of the implantable medical device at a desired location, such as a heart valve annulus, and to provide for ease in the delivery and withdrawal of the delivery system through a patient's vasculature.

As illustrated in FIG. 1A, the catheter portion 102 of the delivery system 100 also preferably includes the outer shaft 108 that is operatively connected with the control handle portion 106. The outer shaft 108 forms an axial lumen that extends along the central longitudinal axis CLA from the control handle portion 106 to the distal portion 104. The axial lumen of the outer shaft 108 surrounds one or more inner shafts, such as an inner shaft 110 (illustrated as dashed lines), over at least a part of its length. The catheter portion 102 can also include an outer stability shaft 109 that covers a portion of the outer shaft 108 at the proximal end of the catheter portion 102. The inner shaft 110 is operatively connected with the control handle portion 106 so as to be movable by operation of the control handle portion 106. In embodiments, an implantable medical device can be coupled to the inner shaft 110 in a compressed (non-expanded) state for delivery to the implant location. The capsule 114 is removably placed over a portion of the implantable medical device. The capsule 114 operates to protect the implantable medical device during delivery to the implant location through a patient's vasculature. The control handle portion 106 can include an adjustable handle control 112 that can be manipulated, e.g., rotated, to deflect the outer shaft 108, the inner shaft 110, or combinations thereof.

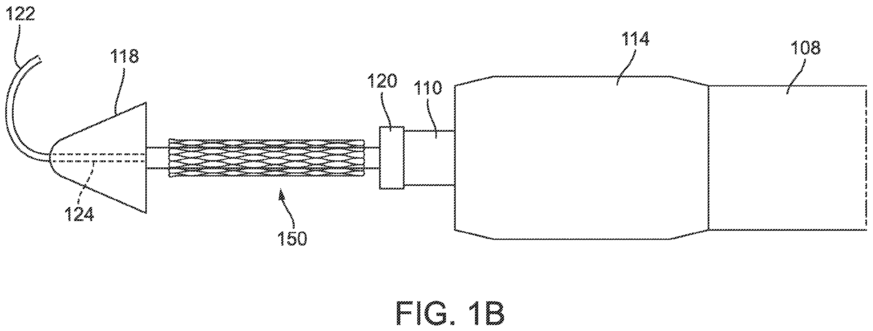

Once the implantable medical device is positioned at the implant location, the capsule 114 can be removed from the implantable medical device, and the implantable medical device can be transitioned from a compressed state to an uncompressed (expanded) state to engage native anatomy at the implant location. For example, the implantable medical device can be loaded over a shaft assembly (not shown) of the distal portion 104 that is coupled to the inner shaft 110. The implantable medical device can be compressively retained within the capsule 114. As illustrated in FIG. 1B, which is an enlarged view of the distal portion 104, the delivery system 100 includes nosecone 118 coupled to a distal end of the inner shaft 110. The delivery system 100 can include an implantable medical device 150 coupled to the inner shaft 110. The inner shaft 110 can include a retention member 120 *(e.g.,* spindle) which is configured to selectively receive corresponding features of the implantable medical device 150 *(e.g.,* paddles, posts, or eyelets). In some embodiments, the capsule 114 can be removed proximally, for example, by attachment to the outer shaft 108 of the catheter portion 102, which can be withdrawn. That is, the outer shaft 108 can be coupled to an actuator (e.g., the adjustable handle control 112) of the control handle portion 106. When the actuator is actuated, the outer shaft 108 and the capsule 114 are retracted proximally relative to the inner shaft 110, thereby uncovering the implantable medical device to allow the implantable medical device to be expanded.

In some embodiments, the implantable medical device can be self-expanding. For example, the implantable medical device can be constructed of a material that transitions from the compressed state to the uncompressed state when the capsule 114 is removed and the implantable medical device is decoupled from shaft assembly of the inner shaft 110. For example, a stent or frame of the implantable medical device can be formed from a shape memory material such as a nickel titanium alloy *(e.g.,* Nitinol) that is self-expandable from the compressed state to the expanded state, such as by the application of heat, energy, and the like, or by the removal of external forces (e.g., compressive forces). In some embodiments, the implantable medical device can be expanded using expansion devices such as a balloon that are coupled to the shaft assembly of the distal portion 104.

The catheter portion 102 can include other components for operation of the delivery system. In some embodiments, as illustrated in FIG. 1B, the inner shaft 110 can also include an axial lumen 124 extending entirely through at least the inner shaft 110, the purpose of which is for receiving a guidewire 122 in order for the delivery system 100 to be guided along a patient's vasculature to an implant location. In some embodiments, the guidewire 122 can be backloaded into the axial lumen 124 via an opening in the nosecone 124. In some embodiments, the guidewire can be introduced to the axial lumen 124 via a guidewire port located on the control handle portion 106. The guidewire, not shown, may be used in a conventional manner to guide the delivery system along it and with its distal end guided to its desired implant location.

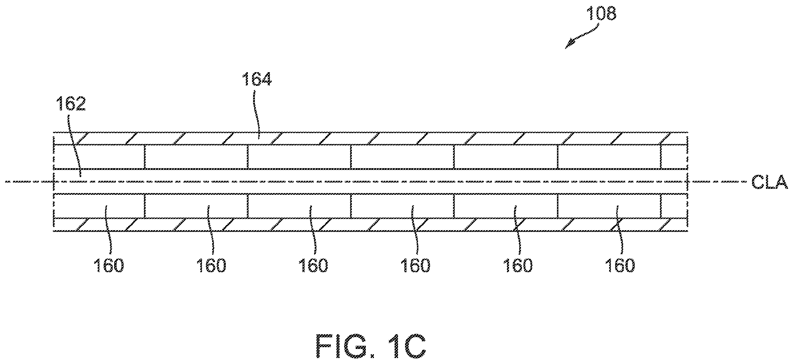

In embodiments, the outer shaft 108 extends from the control handle portion 106 to facilitate the advancement and steering of the delivery system 102 through a patient's vasculature. As illustrated in FIG. 1C, which is cross-sectional view of a portion of the outer shaft 108, the outer shaft 108 is formed of segments 160 that repeat along the central longitudinal axis of the catheter portion 102 from the control handle portion 106 to the distal portion 104. Each of the segments 160 is coupled to or interlocked with an adjacent segment 160 such that each of the segments 160 moves independently relative to one another. That is, adjacent segments 160 are movably coupled so that the outer shaft 108 can move in multiples planes of motion relative to the central longitudinal axis CLA. In some embodiments, the segments 160 can tilt or rotate relative to one another thereby allowing the outer shaft 108 to flex or bend in multiple directions relative to the central longitudinal axis CLA. In some embodiments, the segments 160 can move proximally or distally along the central longitudinal axis CLA thereby allowing the outer shaft 108 to stretch or compress along the central longitudinal axis CLA. In some embodiments, the segments 160 can rotate about the central longitudinal axis CLA. Due to the ability of the outer shaft 108 to bend or flex in multiple planes of motion relative to the central longitudinal axis CLA, the delivery system 100 enables a physician to steer the catheter portion 102 without the need to re-assess and reposition the implantable medical device when tracking to an implant location.

As illustrated in FIG. 1C, the segments 160 can form an axial lumen 162 through which one or more inner shafts (e.g. , the inner shaft 110) may be positioned from the control handle portion 106 to the distal portion 104. In some embodiments, the outer shaft 108 can include an outer layer 164 that surrounds the segments 160. The outer layer 162 can operate as a cover or protective layer for the interlocking segments 160. For example, the outer layer 162 can be constructed of a flexible polymer such as Pebax 7233, Nylon 12, *etc.*

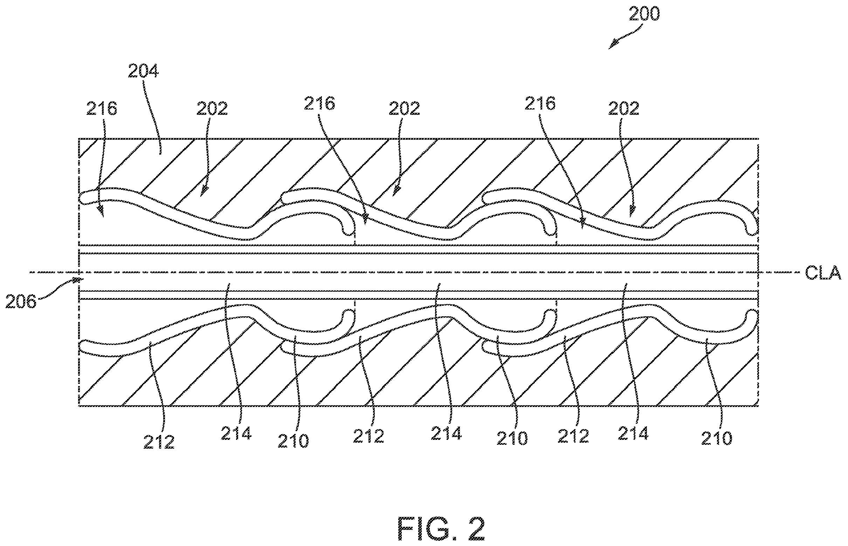

FIG. 2 illustrates an example of an outer shaft 200 with segments in accordance with an embodiment hereof. One skilled in the art will realize that FIG. 2 illustrates one example of a shaft and that existing components illustrated in FIG. 2 may be removed and/or additional components may be added to the outer shaft 200.

As illustrated, the outer shaft 200 includes a plurality of interlocking segments 202 that are arranged along a central longitudinal axis of a catheter of a delivery system, *e.g.,* the catheter portion 102 of the delivery system 100. The outer shaft 200 can also include an outer layer 204 that surrounds the interlocking segments 202. In embodiments, the outer layer 204 can operate as a cover or protective layer for the interlocking segments 202. For example, the outer layer 204 can be constructed of a flexible material such as a flexible polymer. Each of the interlocking segments 202 includes a hollow center portion 214 that extends along the central longitudinal axis CLA from a proximal end to a distal end of each interlocking segment 202. When the interlocking segments are coupled together, the hollow center portions 214 form an axial lumen 206 that extends from a proximal end to a distal end of the outer shaft 200. The axial lumen 206 can be configured to receive one or more inner shafts, such as an inner shaft 110 of the delivery system 100.

In embodiments, the interlocking segments 202 of the outer shaft 200 are configured as interlocking ball and socket or cup segments, which allow the outer shaft 200 to flex or bend in any direction relative to the central longitudinal axis CLA. Each of the interlocking segments 202 includes a ball portion 210 and a socket (or cup) portion 212. The ball portion 210 of one interlocking segment 202 engages with a socket portion 212 of an adjacent interlocking segment 202 thereby forming a repeating length of the interlocking segments 200. In embodiments, the ball portion 210 fits within an inner cavity formed by the socket portion 212 of an adjacent interlocking segment 202. The ball portion 210 of one interlocking segment 202 is moveably coupled within the socket portion 212 of an adjacent interlocking segment 202 such that ball portion 210 can move within an inner cavity 216 of the socket portion 212. As such, the interlocking segments 202 can move relative to one another, for example, tilt relative to one another and/or rotate about the central longitudinal axis relative to one another. In any embodiment, the outer shaft 200 formed of interlinking segments 202 can facilitate uniform and increased flexibility in the outer shaft 200, which improves tracking and steering a catheter (e.g., the catheter portion 102 of the delivery system 100) through tortuous anatomy.

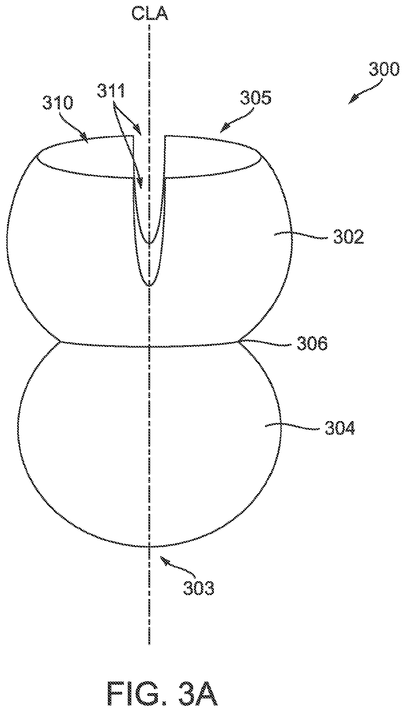

FIGS. 3A-3G illustrate one example of an interlocking segment 300 that can be used in the outer shaft 200 in accordance with an embodiment hereof. One skilled in the art will realize that FIGS. 3A-3G illustrate one example of an interlocking segment and that existing components illustrated in FIGS. 3A-3G may be removed and/or additional components may be added to the interlocking segment 300.

As illustrated in FIG. 3A, which is a perspective view of the interlocking segment 300, the interlocking segment 300 includes a socket (or cup) 302 and a ball 304. The socket 302 is coupled to the ball 304 by a neck 306. In embodiments, the interlocking segment 300 can be constructed as a solid body that has a channel 310. The channel 310 is formed along a longitudinal axis (LA) of the interlocking segment 300 from a first end 303 to a second end 305 of the interlocking segment 300. The socket 302 includes windows 311 formed on opposing sides of the socket 302. In embodiments, the windows 311 are formed as cut-outs or open spaces in opposing sides of the socket 302. The windows 311 operate to allow adjacent interlocking segments 300 to be moveable coupled as described below. In embodiments, the windows 311 can be constructed to any shape and size that has a width that tapers from the distal end 305 towards the proximal end 303, as illustrated. For example, the windows 311 can be constructed having a triangular shape with linear or curved sides.

**In** some embodiments, when installed on a delivery system (e.g., the delivery system 100), the outer shaft 200 can be oriented such that the first end 303 of each interlocking segment 300 is orientated towards the proximal end of the delivery system 100. In some embodiments, when installed on a delivery system (e.g., the delivery system 100), the outer shaft 200 can be oriented such that the second end 305 of each interlocking segment 300 is orientated towards the proximal end of the delivery system 100. In embodiments, the socket 302, the ball 304, and the neck 306 can be constructed of any material that provides structural support to an outer shaft, such as the outer shaft 200. For example, the socket 302, the ball 304, and the neck 306 can be formed of metal, metal alloys, polymeric material, *etc.*

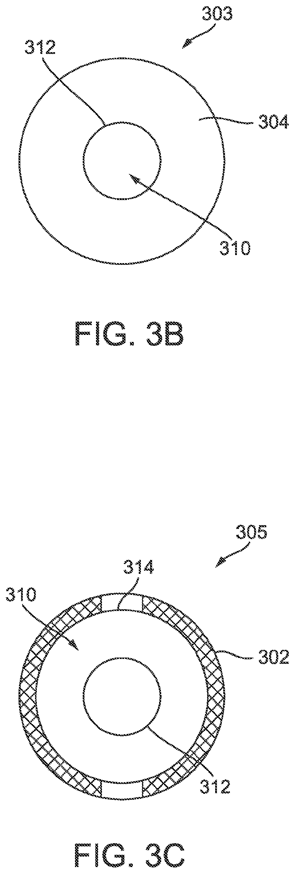

As illustrated in FIG. 3B, which is a view of the first end 303 of the interlocking segment 300, the ball 304 includes an opening 312 that is positioned at the first end 303 of the interlocking segment 300. The opening 312 is coupled to a proximal end of the channel 310 thereby defining a first opening of the channel 310. As illustrated in FIG. 3C, which is a view of the second end 305 of the interlocking segment 300, the socket 302 includes an opening 314 positioned at the second end 305 of the interlocking segment 300. The opening 314 is coupled to a second end of the channel 310 thereby defining a second opening of the channel 310.

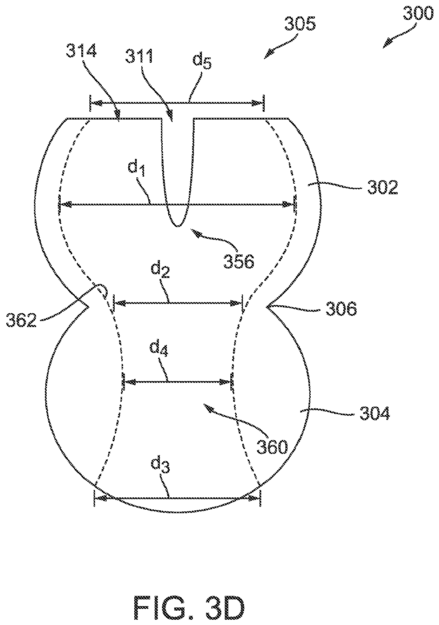

FIG. 3D illustrates a cross-sectional view of the interlocking segment 300, which is taken along the longitudinal axis LA of the interlocking segment 300. As illustrated, the socket 302 can be constructed having a hollow spherical shape thereby defining a socket cavity 356. includes a first portion 350 and a second portion 352. The socket cavity 356 include an opening 314 formed at the distal end 305 of the socket 302. In embodiments, the socket cavity 356 can form a distal portion of the channel 310. In embodiments, the socket 302 can be constructed having a maximum inner diameter, di₁, *(e.g.,* an outer diameter of the socket cavity 356) that allows a ball 304 of another interlocking segment 300 to fit within the socket cavity 356. For example, the diameter, d₁, can be a diameter that allows coupling with a ball 304 of another interlocking segment 300 to fit within the socket cavity 356. The socket 302 can be constructed having a distal inner diameter, d₅, *(e.g.,* the outer diameter of the opening 314) that allows maintain a ball 304 of an adjacent interlocking segment 300 with the socket cavity 356. For example, the socket 302 can be constructed having a distal inner diameter, d₅, that is larger than the diameter of the neck 306. In embodiments, a diameter of the opening 314 can that is less than the diameter, d₁.

The ball 304 can be constructed having a spherical shape with a circular cross-section. A ball cavity 360 can be formed through the ball 304 that extends from the opening 312 at the first end 303 to an opening 362 at a connection between the ball 304 and the neck 306. The opening 362 opens into the socket cavity 356, thereby forming the channel 310 as the combination of the bally cavity 360 and the socket cavity 356. As illustrated, the ball cavity 360 can be constructed having a hollow shape that curves inward at a midpoint between the opening 312 and the opening 362, *e.g.,* a circular hyperboloid shape. The ball cavity 360 forms a proximal portion of the channel 310. In embodiments, the opening 362 of the ball cavity 360 can be constructed having a diameter that corresponds to the second inner diameter, d₂, located at the proximal end of the second portion 352. In embodiments, the opening 312 can be constructed having a diameter, d₃, that accommodates movement of the ball 304 within a socket cavity 356 of a socket 302 of an adjacent interlocking segment 300 without interfering with an inner shaft contained within the ball cavity 360, as described below in further detail. In some embodiments, the diameter, d₃, can be approximately equal to the second inner diameter, d₂, of the second portion 352 of the socket 302. The ball cavity 360 can also be constructed having a minimum inner diameter, d₄, that allows one or more inner shafts (e.g., the inner shaft 110) to pass through the ball cavity 360. In some embodiments, the minimum inner diameter, d₄, can correspond to an approximate midpoint of the ball cavity 360.

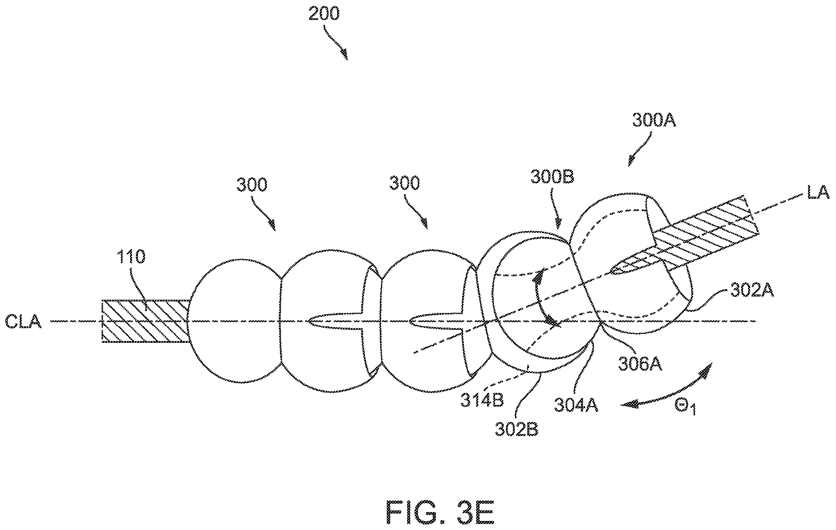

As illustrated in FIG. 3E, the interlocking segments 300 can be coupled together in a repeating pattern (e.g., a ball 304 of one interlocking segment 300 positioned within a socket 302 of an adjacent interlocking segment 300) to form a shaft 200 *(e.g.,* the outer shaft 108 of the delivery system 100). While FIG. 3E illustrates the shaft 200 without the outer layer 204, one skilled in the art will realize that the shaft 200 including the interlocking segments 300 can include an outer layer 204. When coupled together in a repeating arrangement, the channels 310 of the interlocking segments 300 can form the axial lumen 206. As discussed above, the axial lumen 206 can receive one or more inner shafts such as the inner shaft 110, as illustrated in FIG. 3E.

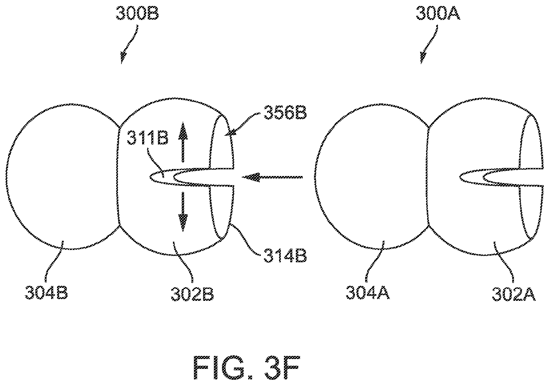

**In** some embodiments, as illustrated in FIG. 3F, the interlocking segments 300 (e.g., interlocking segment 300A and interlocking segment 300B) can be configured to couple to adjacent interlocking segments 300 by snap fit. As illustrated in FIG. 3F, to couple the interlocking segment 300A and the interlocking segment 300B, the ball 304A of the interlocking segment 300A can be inserted into the socket 302B of the interlocking segment 300B. Because an outer diameter of the ball 304A is larger than the diameter of the opening 314B, the ball 304A applies a force on sides of the socket 302B on either side of the windows 311B as the ball 304A is inserted through the opening 314B. The force causes the sides of the socket 302B to move outward at a pivot point of the windows 311B, thereby enlarging the opening 314B. As such, the ball 304A can be inserted into the socket cavity 356B. Once the ball 304A enters the socket cavity 356B, the force is removed, and the sides of the socket 302B move inward, thereby reducing the diameter of the opening 314B. As such, the ball 304A of the interlocking segment 300A is contained within the socket cavity 356B. Moreover, as discussed below, the ball 304 can move within the socket cavity 356B because the socket cavity 356B has a diameter and a volume that is larger than the ball 304A.

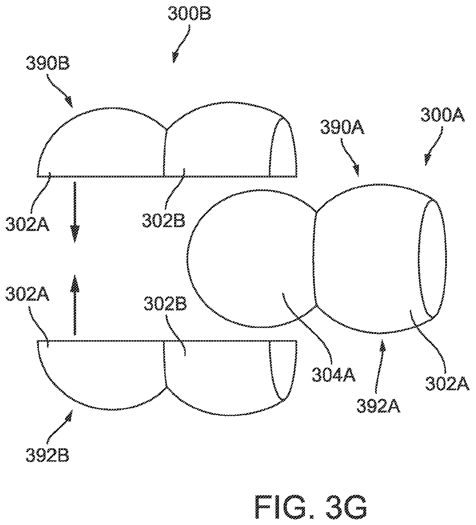

In some embodiments, as illustrated in FIG. 3G, each of the interlocking segments 300 (e.g., interlocking segment 300A and interlocking segment 300B) can be constructed as two separate pieces, 390 and 392, that can be separated in order to couple to adjacent interlocking segments 300. For example, the interlocking segment 300B can include a first piece 390B and a second piece 392B that are removably coupled and can separate along the longitudinal axis. In embodiments, the first piece 390B and the second piece 392B can be removably coupled using any type of mechanical connection, e.g., snap fit, friction fit, *etc.,* or non-removable, non-mechanical connection such as adhesive, welding, *etc.* To couple the interlocking segment 300A to the interlocking segment 300B, the first piece 390B can be separated from the second piece 392B, thereby opening the socket cavity 356B. Then, the ball 304A can be aligned in the socket cavity 356B between the first piece 390B and second piece 392B. The first piece 390B and the second piece 392B can be rejoined, thereby containing the ball 304A of the interlocking segment 300A within the socket cavity 356B. The ball 304 can move within the socket cavity 356B because the socket cavity 356B has a diameter and a volume that is larger than the ball 304A.

In embodiments, when coupled, the ball 304 of one interlocking segment 300 can move (e.g., tilt and rotate) within the socket cavity 356 of the socket 302 of an adjacent interlocking segment 300. As such, each interlocking segment 300 can move in multiple planes of motion relative to each other. For example, as illustrated in FIG. 3E, an interlocking segment 300A can tilt relative to an interlocking segment 300B. Because the ball 304A is spherically shaped and the opening 314B is circular, the interlocking segment 300A can tilt in any direction relative the longitudinal axis of the interlocking segment 300B. The interlocking segment 300A can tilt until the neck portion 306A contacts the second end 305 of the interlocking segment 300B *(e.g.,* an edge of the socket cavity 356 of the socket 302 forming the opening 314). As such, the interlocking segment 302A can tilt, relative to the central longitudinal axis, to a maximum angle, θ₁. In embodiments, the maximum angle, θ₁, can depend on dimensions of the interlocking segments, e.g., the diameter of the opening 314, the diameter of the neck 306, *etc.,* and can be selected based on the requirements of the outer shaft, *e.g.,* bend radius, inner lumen diameter, *etc.* For example, the maximum angle, θ₁, can range between approximately 10 degrees to approximately 45 degrees. Additionally, because the ball 304A is free to move within the socket cavity 356 of the socket 302B, the interlocking segment 300A can rotate about the longitudinal axis of the interlocking segment 300A. While the above motion is discussed with reference to interlocking segment 300A and 300B, one skilled in the art will realize that similar relative motion can occur in any of the interlocking segments 300.

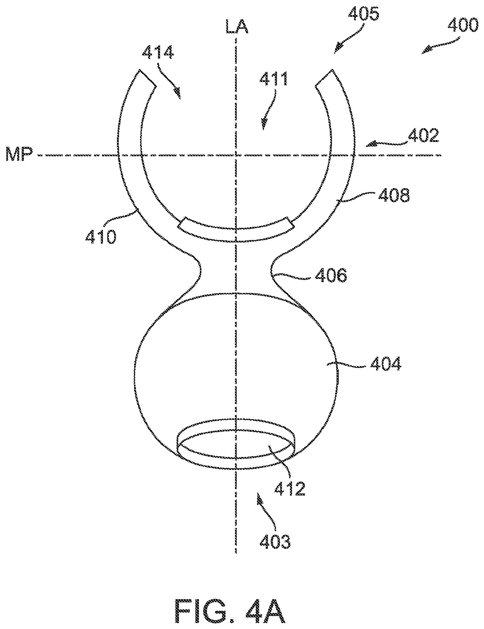

FIGS. 4A-4G illustrate another example of an interlocking segment 400 that can be used in the outer shaft 200 in accordance with an embodiment hereof. One skilled in the art will realize that FIGS. 4A-4G illustrate one example of an interlocking segment and that existing components illustrated in FIGS. 4A-4G may be removed and/or additional components may be added to the interlocking segment 400.

As illustrated in FIG. 4A, which is a perspective view of the interlocking segment 400, the interlocking segment 400 includes a socket 402 and a ball 404. The socket 402 is coupled to the ball 404 by a neck 406. The socket 402 includes a leg 408 and a leg 410 that are positioned on opposing sides of the socket 402. The leg 408 and the leg 410 define a socket cavity 411 that has a second opening 414 at a second end 405 of the interlocking segment 400. The leg 408 and the leg 410 extend outward from the neck 406 in a direction along a longitudinal axis (LA) towards the second end 405 of the interlocking segment 400. Additional, the leg 408 and the leg 410 curve outward away from a longitudinal axis from a connection with the neck 410 to a midpoint (MP) of the socket 402 and curve back inward towards the longitudinal axis from the midpoint to the second end 405. That is, the leg 406 and leg 410 form opposing arcs from the connection of the socket 402 to the second end 405. As such, the leg 408 and the leg 410 define the socket cavity 411 having an open and hollow spherical shape.

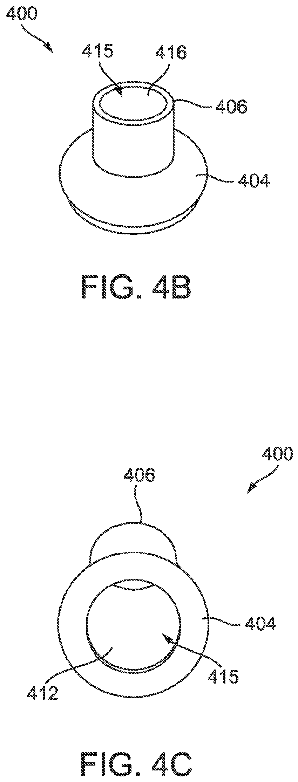

In embodiments, as illustrated in FIGS. 4B and 4C, which are perspective views of the interlocking segment 400 with the leg 408 and the leg 410 removed, the ball 404 and the neck 406 of the interlocking segment 400 can be constructed as a solid integrated body that has a channel 415 formed therein. The channel 415 is formed along a longitudinal axis (LA) of the ball of the interlocking segment 400 from a first end 403 of the interlocking segment 400 to a distal end of the neck 406. The ball 404 includes a first opening 412 that forms a first opening of the channel 415. The neck 406 includes a second opening 416 that forms a second opening of the channel 415 into the socket cavity 411. In some embodiments, when installed on a delivery system *(e.g.,* the delivery system 100), the outer shaft 200 can be oriented such that the first end 403 of each interlocking segment 400 is orientated towards the proximal end of the delivery system 100. In some embodiments, when installed on a delivery system (e.g., the delivery system 100), the outer shaft 200 can be oriented such that the second end 405 of each interlocking segment 400 is orientated towards the proximal end of the delivery system 100. In embodiments, the socket 402, the ball 404, and the neck 406 can be constructed of any material that provides structural support to an outer shaft, such as the outer shaft 200. For example, the socket 402, the ball 404, and the neck 406 can be formed of metal, metal alloys, polymeric material, and *etc.*

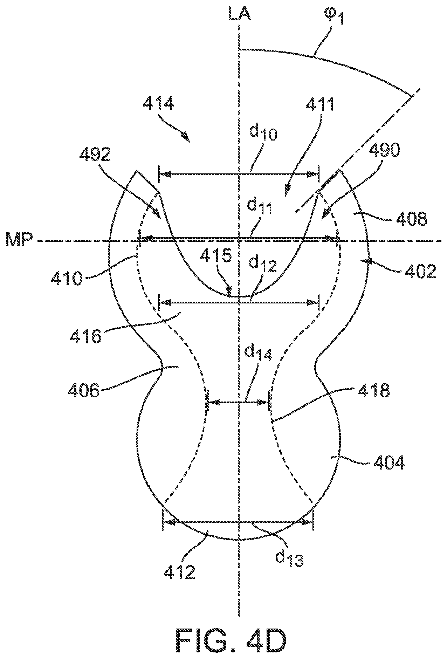

FIG. 4D illustrates a cross-sectional view of the interlocking segment 400, which is taken along the longitudinal axis of the interlocking segment 400. As illustrated, the legs 408 and the leg 410 of the socket 402 can be constructed having a distal inner diameter, d₁₀, *(e.g.,* an outer diameter of the socket cavity 411 at the second opening 414). Additionally, the legs 408 and the leg 410 of the socket 402 can be constructed having a midpoint diameter, d₁₁, *(e.g.,* a maximum outer diameter of the socket cavity 411 at the midpoint). The midpoint diameter, d₁₁, can be formed to a diameter that allows a ball 404 of another interlocking segment 400 to fit within the socket cavity 411. As illustrated in FIG. 4D, the leg 408 and the leg 410 extend circumferentially (e.g., in the plane perpendicular to the longitudinal axis, LA, of the interlocking segment 400). As such, the leg 408 and the leg 410 form spherical-shaped pockets 490 and 492, respectively. When a ball 404 of an adjacent interlocking segment 400 is inserted into the socket cavity 411, the pocket 490 and the pocket 492 *(e.g.,* the circumferentially extension of the leg 408 and leg 410) create an overlap between the ball 404 of an adjacent interlocking segment 400 and the leg 408 and the leg 410, thereby maintaining the ball 404 within the partially open socket cavity 411. Additionally, the legs 408 and the leg 410 of the socket 402 can be constructed having an inner diameter, d₁₂, *(e.g.,* an outer diameter of the socket cavity 411 at the connection with the neck 416).

The ball 404 can be constructed having a spherical shape with a circular cross-section. The neck 406 can be constructed having a cylindrical shape with a circular cross section. The channel 415 can be formed through the ball 404 and the neck 404, extending from the first opening 412 in the first end 403 to the second opening 416 at a connection between the socket 402 and the neck 406. As illustrated, the channel 415 can be constructed having a hollow shape that curves inward at a midpoint between the first opening 412 and the second opening 416, *e.g.,* a circular hyperboloid shape. The second opening 416 of the channel 415 can coupled the distal cavity 412 to the channel 415. In embodiments, the second opening 416 of the channel 415 can be constructed having a diameter that corresponds to the inner diameter, d₁₂, located at the connection point of the socket 402 and the neck 406. In embodiments, the first opening 412 can be constructed having a diameter, d₁₃, that accommodates movement of the ball 404 within a socket 402 of an adjacent interlocking segment 400 without interfering with inner shafts contained within the channel 415, as described below in further detail. In some embodiments, the diameter, d₁₃, can be approximately equal to the inner diameter, d₁₂. In some embodiments, the diameter, d₁₃, can be approximately larger than the inner diameter, d₁₂. The channel 415 can also be constructed having a minimum inner diameter, d₁₄, that allows one or more inner shafts (*e.g.,* the inner shaft 110) to pass through the channel 415. In some embodiments, the minimum inner diameter, d₁₄, can correspond to an approximate midpoint of the channel 415. For example, the minimum inner diameter, d₁₄, can range from approximately 2.50 mm to approximately 3.50 mm, the midpoint diameter, d₁₁, can range from approximately 4.0 mm and 5.0 mm. Further, for example, inner diameter, d₁₀, the inner diameter, d₁₂, and the diameter, d₁₃, can range from approximately 2.60 mm to approximately 4.9 mm. One skilled in the art will realize that any examples of dimensions describe herein are approximate values and can vary by, for example, +/- 5.0 %, based on manufacturing tolerances, operating conditions, and/or other factors.

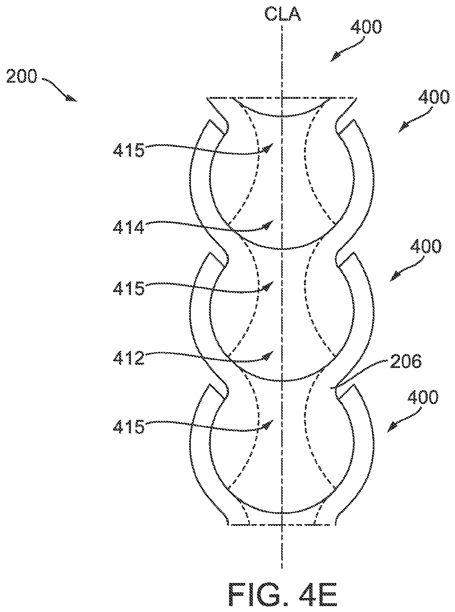

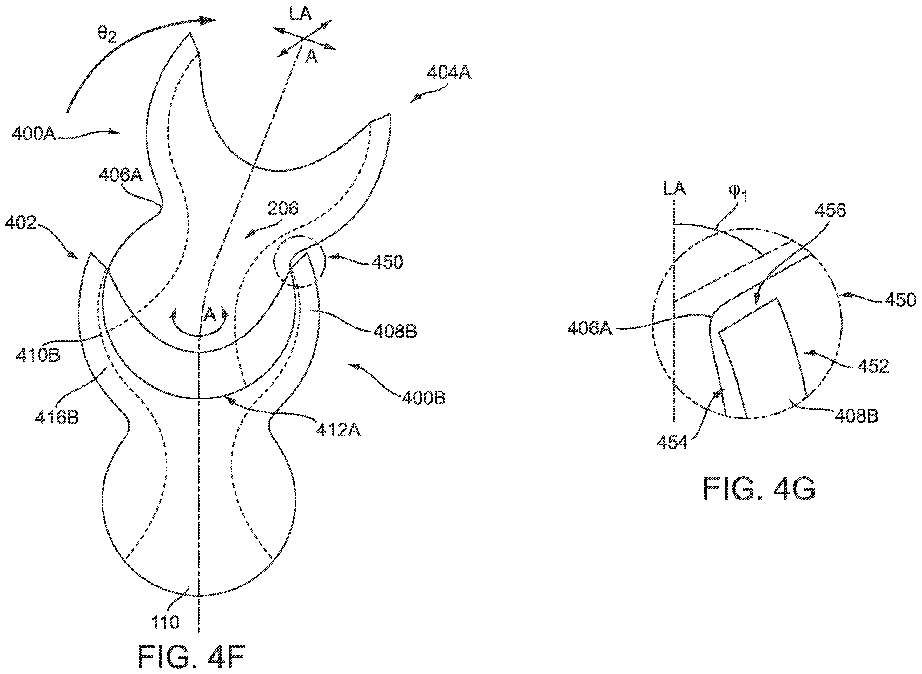

As illustrated in FIG. 4E and 4F, which are cross-section views, the interlocking segments 400 can be coupled together in a repeating pattern (e.g., a ball 404 of one interlocking segment 400 positioned with a socket 402 of an adjacent interlocking segment 400) to form a shaft 200 *(e.g.,* the outer shaft 108 of the delivery system 100). While FIG. 4E and 4F illustrates the shaft 200 without the outer layer 204, one skilled in the art will realize that the shaft 200 including the interlocking segments 400 can include an outer layer 204. In some embodiments, the interlocking segments 400 (e.g., interlocking segment 400A and interlocking segment 400B) can be configured to couple to adjacent interlocking segments 400 by snap fit. For example, as similarly described above with reference to 3F, to couple the interlocking segment 400A and the interlocking segment 400B, the ball 404A of the interlocking segment 400A can be inserted into the socket 402B of the interlocking segment 400B. Because an outer diameter of the ball 404A is larger than the diameter of the opening 414B, the ball 404A applies a force on the leg 408B and the leg 410B as the ball 404A is inserted through the opening 414B. The force causes the leg 408B and the leg 410B of the socket 402B to move outward, thereby enlarging the opening 414B. As such, the ball 404A can be inserted into the socket cavity 411B. Once the ball 404A enters the socket cavity 411B, the force is removed, and the leg 408B and the leg 410B of the socket 402B move inward, thereby reducing the diameter of the opening 414B. As such, the ball 404A of the interlocking segment 400A is contained within the socket cavity 411B, e.g., the pockets 490 and 492. Moreover, as discussed below, the ball 404 can move within the socket cavity 411B because the socket cavity 411B has a diameter and a volume that is larger than the ball 404A.

When coupled together in a repeating arrangement, the channels 415 of the interlocking segments 400 can form the axial lumen 206. As discussed above, the axial lumen 206 can receive one or more inner shafts such as the inner shaft 110, as illustrated in FIG. 4F. When coupled, the ball 404 of one interlocking segment 400 can move (e.g., tilt and rotate) within the socket 402 of an adjacent interlocking segment 400. As such, each interlocking segment 400 can move in multiple planes of motion relative to each other. For example, as illustrated in FIG. 4F, an interlocking segment 400A can tilt relative to an interlocking segment 400B. Because the ball 404A is spherically shaped, the interlocking segment 400A can tilt in any direction relative the longitudinal axis of the interlocking segment 400B (and/or the central longitudinal axis of the outer shaft 200. As discussed above, the first opening 412 at the first end 403 and the second opening 416 at the neck 406 can include enlarged diameters relative to the minimum diameter of the channel 415. As illustrated in FIG. 4F, as the interlocking segment 400A tilts relative to the interlocking segment 400B, a portion of the first opening 412A becomes blocked by one of the legs, *e.g.,* leg 410A or leg 410B. In embodiments, the first opening 412 can be constructed having a diameter, d₁₃, such that, when the interlocking segment 400B tilts relative to the interlocking segment 400A to a maximum tilt angle, θ₂, the first opening 412 remain open to allow access to the channel 415. As such, the channel 415A and the channel 415B remain coupled thereby maintaining the axial lumen 206. That is, the first opening 412A of the interlocking segment 400A and the second opening 416B of the interlocking segment 400B do not interfere (e.g., bind, crimp, *etc.*) with the inner shaft 110 when the interlocking segment 400A tilts.

In embodiments, the interlocking segment 400A can tilt until the neck portion 406A contacts a distal end 450 of the interlocking segment 400B *(e.g.,* a slanted end 456 of the leg 408A). That is, as illustrated in FIG. 4G, which is an enlarged view of the distal end 450 of the leg 408A, the leg 408A can included the slanted end 456 formed between an outer surface 452 and an inner surface 454 of the leg 408A. The slanted end 456 can be slanted at an angle, φ₁, relative to the longitudinal axis, LA, of an interlocking segment 400, as illustrated in FIG. 4D, for example, between the inner surface 454 and the outer surface 452 as illustrated in FIG. 4G. The angle, φ₁, of the slanted end 456 defines a maximum tilt angle, θ₂, of the interlocking segment 400A, as illustrated in FIG. 4F. If the slanted end 456 is constructed having a larger angle, φ₁, the neck 406A of the interlocking segment 400A contacts the slanted end 456 at lower tilt angle, θ₂, relative to the longitudinal axis of the interlocking segment 400B. Additionally, because the ball 404A is free to move within the socket 402B, the interlocking segment 400A can rotate about the longitudinal axis of the interlocking segment 400A. In embodiments, the maximum angle, θ₂, can depend on dimensions of the interlocking segments, e.g., title angle φ₁, the diameter of the neck 406, *etc.,* and can be selected based on the requirements of the outer shaft, *e.g.,* bend radius, inner lumen diameter, *etc.* For example, the maximum angle, θ₂, can range between approximately 10 degrees to approximately 45 degrees. While the above motion is discussed with reference to interlocking segments 400A and 400B, one skilled in the art will realize that similar relative motion can occur in any of the interlocking segments 300.

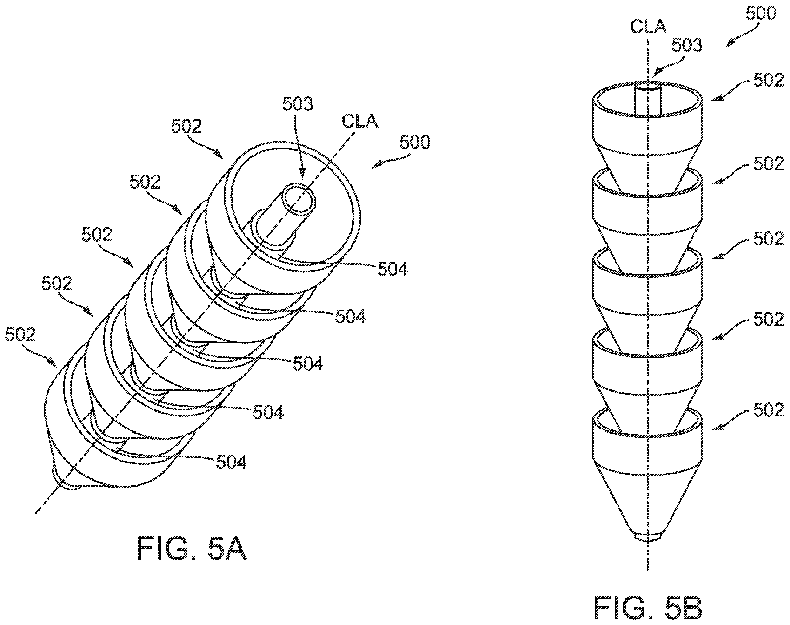

FIGS. 5A-5E illustrate another example of an outer shaft 500 that can be used in the delivery system 100 in accordance with an embodiment hereof. One skilled in the art will realize that FIGS. 5A-5E illustrate one example of a shaft and that existing components illustrated in FIGS. 5A-5E may be removed and/or additional components may be added to the outer shaft 500.

As illustrated in FIGS. 5A and 5B, which are perspective views of the outer shaft 500, the outer shaft 500 includes a repeating arrangement of funnel segments 502. The repeating arrangement of the funnel segments 502 forms a non-continuous backbone that allow flexibility in multiple planes of motion. The outer shaft 500 also include an inner shaft 503 that runs along the central longitudinal axis, CLA, through the center of the funnel segments 502. The inner shaft 503 can form a lumen through which one or more additional inner shafts can be placed, e.g., the inner shaft 110 of the delivery system 100. In some embodiments, the inner shaft 503 can be formed of a braided material. In embodiments, the funnel segments 502 can be constructed of any material that provides structural support to the outer shaft 500. For example, the funnel segments 502 can be formed of metal, metal alloys, polymeric material, and *etc.*

The outer shaft 500 also includes washers 504 that are positioned between adjacent funnel segments 502. The washers 504 are fixed to and surround the inner shaft 503 between adjacent funnel segments 502 and operate as shock absorbers when adjacent funnel segments 502 are compressed. The funnel segments 502 can be moveably coupled to the inner shaft 503 thereby allowing the funnel segments 502 to rotate around the inner shaft. Likewise, the funnel segments 502 can move in the direction of the central longitudinal axis along the inner shaft 503 with the washers 504 acting as stops for the funnel segments 502. As such, the funnel segments 502 can move longitudinally along the inner shaft 503 within a limit set by the washers 504 thereby providing strength when the washers 504 are set closer to each other and flexibility when the washers 504 are more separated from each other. In embodiments, the washers 504 can be constructed of any material that provides support and shock absorption for the funnel segments 502. For example, the washers 504 can be formed of metal, metal alloys, polymeric material, and *etc.* In some embodiments, the inner shaft 503 can be coated with a lubricious coating to reduce the friction between the funnel segments 502 and the inner shaft 503 when the funnel segments 502 move relative to the inner shaft 503.

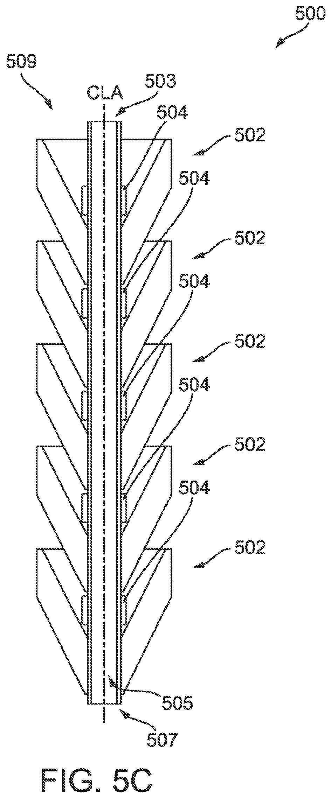

As illustrated in FIG 5C, which is a cross-sectional view of the outer shaft 500 taken along the central longitudinal axis, the inner shaft 503 defines an axial lumen 505 that extends from a first end 507 to a second end 509 of the outer shaft 500. The axial lumen 505 can be configured to receive one or more inner shafts, such as an inner shaft 110 of the delivery system 100. The outer shaft 500 can also include an outer layer (not shown) that surrounds the funnel segments 502. In embodiments, the outer layer can operate as a cover or protective layer for the funnel segments 502. For example, the outer layer can be constructed of a flexible material such as a flexible polymer.

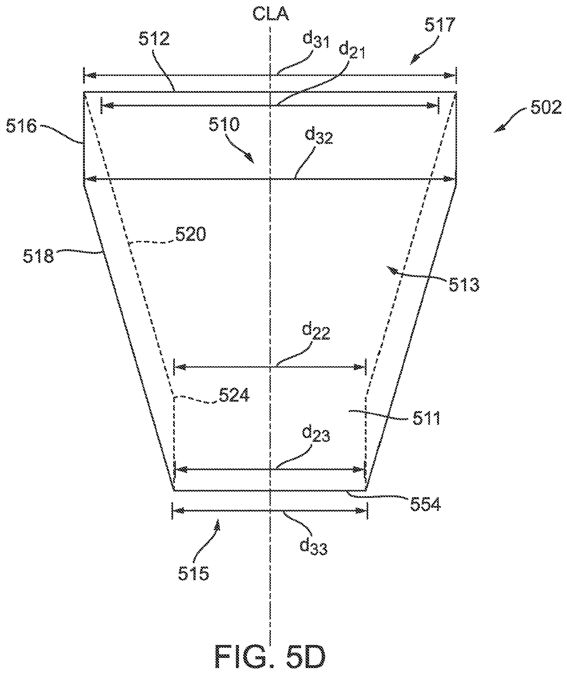

FIG. 5D illustrates a cross-section view of a funnel segment 502 taken along a longitudinal axis of the funnel segment 502. The funnel segment 502 can be constructed as a solid body having a channel 510 formed therein. The channel 510 is formed along a longitudinal axis of the funnel segment 502 from a first end 515 to a second end 517 of the funnel segment 502. The funnel segment 502 includes a first opening 514 that is positioned at the first end 515 of the funnel segment 502. The first opening 514 is coupled to a first end of the channel 510 thereby defining a first opening of the channel 510. The funnel segment 502 includes a second opening 512 positioned at the second end 517 of the funnel segment 502. The second opening 512 is coupled to a second end of the channel 510 thereby defining a second opening of the channel 510.

In embodiments, the channel 510 includes a first portion 511 formed by a first inner sidewall 524 of the funnel segment 502. The channel 510 also includes a first portion 511 formed by a second inner sidewall 520 of the funnel segment 502. The first portion 511 of the channel 510 is positioned proximate to the first end of the funnel segment 502 and is coupled to the first opening 514. The first portion 511 of the channel 510 can be constructed having a hollow cylindrical shape. In embodiments, the first inner sidewall 524 that forms the first portion 511 of the channel 510 can be constructed having an inner diameter, d₂₃, that movement of the funnel segments 502 along the inner shaft 503. For example, the diameter, d₂₃, can be a diameter that is slightly larger than an outer diameter of the inner shaft 503.

The second portion 513 of the channel 510 is positioned proximate to the second end 517 of the funnel segment 502 and is coupled to the second opening 512. The second portion 515 of the channel 510 can be constructed having a hollow frustoconical shape, e.g., decreases in diameter from the second opening 512 to a connection with the first portion 511 of the channel 510. In embodiments, the second inner sidewall 520 that forms the distal portion 511 of the channel 510 can be constructed having a first inner diameter dimeter, d₂₁, (e.g., maximum outer diameter of the distal portion 511 of the channel 510) and having a second inner diameter, d₂₂, located at connection with the first inner sidewall 524 *(e.g.,* minimum outer diameter of the distal portion 511 of the channel 510). In embodiments, second inner diameter, d₂₂, can be approximately equal to the diameter, d₂₃.

In embodiments, the funnel segment 502 includes a second outer sidewall 516 positioned proximal to the second end 517. The funnel segment 502 also includes a first outer sidewall 518 positioned proximate to the first end 515. The second outer sidewall 516 can be constructed having a cylindrical shape with a circular cross-section. In embodiments, the second outer sidewall 516 can be constructed having an outer diameter, d₃₁. The first outer sidewall 518 can be constructed having a frustoconical shape, e.g., decreases in diameter from a connection with the second outer sidewall 516 to the first end 515 of the funnel segment 502. In embodiments, the first outer sidewall 518 can be constructed having a first outer diameter dimeter, d₃₂, and having a second outer diameter, d₃₃, located at connection at the first end 515. In embodiments, outer diameter, d₃₁, can be approximately equal to the first outer diameter dimeter, d₃₂. In embodiments, the second outer diameter, d₃₃, of the proximal sidewall 518 can be less than the first inner diameter dimeter, d₂₁, so that an adjacent funnel section 502 can interlock as illustrated in FIG. 5A-5C.

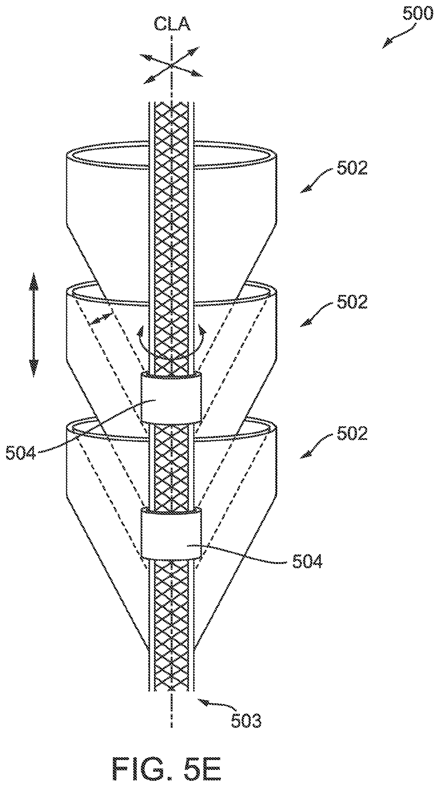

As illustrated in FIG. 5E, when coupled, the funnel segments 502 of outer shaft 500 can move (e.g., tilt and rotate) relative to one other. As such, each funnel segment 502 can move in multiple planes of motion relative to each other. For example, one funnel segment 502 can tilt relative to an adjacent funnel segment 502. Additionally, because the funnel segments 502 are not fixed to the inner shaft 503, the funnel segments 502 can rotate about the central longitudinal axis of the shaft 500. Likewise, the funnel segments 502 can move in the direction of the central longitudinal axis CLA along the inner shaft 503 with the washers 504 acting as stops for the funnel segments 502. As such, the funnel segments 502 can move longitudinally along the inner shaft 503 within a limit set by the washers 504, thereby providing strength when the washers 504 are set are closer to each other and flexibility when the washers are set more separated from each other.

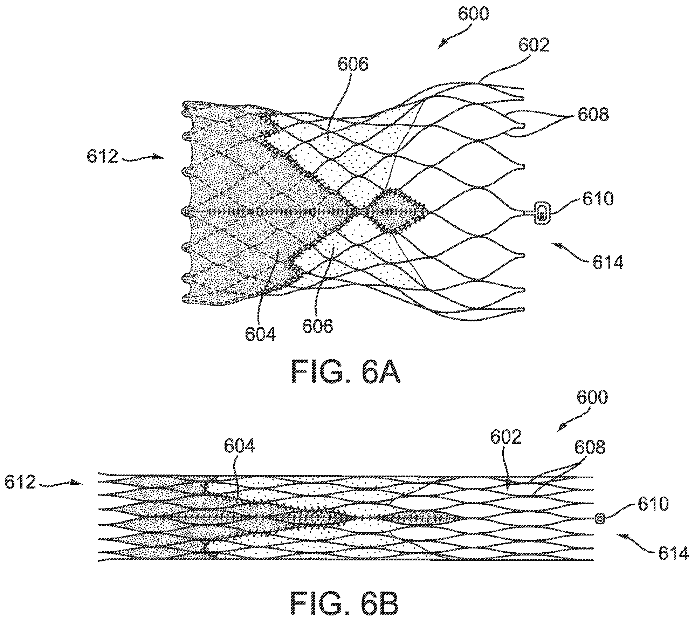

In embodiments, the implantable medical devices useful with the present disclosure can be a prosthetic valve sold under the trade name CoreValve^{®} available from Medtronic, Inc, Evolut^{™} Pro+ available from Medtronic, Inc., and *etc.* A non-limiting example of an implantable medical device, for example, the implantable medical device 150, useful with systems, devices and methods of the present disclosure is illustrated in FIGS. 6A and 6B. In particular, FIG. 6A illustrates a side view of a prosthetic heart valve 600 in a normal or expanded (uncompressed) arrangement. FIG. 6B illustrates the prosthetic heart valve 600 in a compressed arrangement (e.g., when compressively retained within delivery system such as the distal portion 104 of the delivery system 100). The prosthetic heart valve 600 includes a stent or frame 602 and a valve structure 604. The stent 602 can assume any of the forms described above, and is generally constructed so as to be expandable from the compressed arrangement (FIG. 6B) to the uncompressed arrangement (FIG. 6A). In some embodiments, the stent 602 is self-expanding. In other embodiments, the stent 602 is designed to be expanded to the expanded arrangement by a separate device *(e.g.,* a balloon internally located within the stent 602). The valve structure 604 is assembled to the stent 602 and provides two or more (typically three) leaflets 606. The valve structure 604 can be assembled to the stent 602 in various manners, such as by sewing the valve structure 604 to one or more of the wire segments or commissure posts defined by the stent 602.

The prosthetic heart valve 600 of FIGS. 6A and 6B can be configured to replace or repair an aortic valve. Alternatively, other shapes are also envisioned, adapted to the specific anatomy of the valve to be repaired (e.g., stented prosthetic heart valves in accordance with the present disclosure can be shaped and/or sized for replacing a native mitral, pulmonic, or tricuspid valve). With the example of FIGS. 6A and 6B, the valve structure 604 extends less than the entire length of the stent 602, but in other embodiments can extend along an entirety, or a near entirety, of a length of the stent 602. A wide variety of other constructions are also acceptable and within the scope of the present disclosure. For example, the stent 602 can have a more cylindrical shape in the normal, expanded arrangement.

The stent 602 includes support structures that comprise a number of struts or wire portions 608 arranged relative to each other to provide a desired compressibility and strength to the valve structure 604. The stent 602 can also include one or more paddles 610 that removably couple the prosthetic heart valve 600 to a delivery system, e.g., the delivery system 100. While FIGS. 6A and 6B illustrate paddles 610, one skilled in the art will realize that the paddles 610 can be replaced with other components such as eyelets, loops, slots, or any other suitable coupling member. The paddles 610 (or other portions of the stent 602) can include one or more radiopaque markers that aid in the positioning and orientation of the prosthetic heart valve 600. The struts or wire portions 608 form a lumen having an inflow end 612 and an outflow end 614. The struts or wire portions 608 can be arranged such that the struts or wire portions 608 are capable of transitioning from the compressed arrangement to the uncompressed arrangement. These wires are arranged in such a way that the stent 602 allows for folding or compressing or crimping to the compressed arrangement in which the internal diameter is smaller than the internal diameter when in the uncompressed arrangement. In the compressed arrangement, such the stent 602 with attached valve structure 604 can be mounted onto a delivery system, such as the distal portion 104 the delivery system 100. The stent 602 are configured so that they can be changed to an uncompressed arrangement when desired, such as by the relative movement of one or more sheaths relative to a length of the stent 602.

In embodiments, the struts or wire portions 608 of the stent 602 can be formed of a metal or other material that can be expanded from a compressed arrangement to an uncompressed arrangement by an expansion device, e.g., balloon. In some embodiments, the wires of the support structure of the stent 602 in embodiments of the present disclosure can be formed from a shape memory material such as a nickel titanium alloy (e.g., Nitinol). With this material, the support structure is self-expandable from the compressed arrangement to the normal, expanded arrangement, such as by the application of heat, energy, and the like, or by the removal of external forces (e.g., compressive forces). The stent 602 can also be compressed and re-expanded multiple times without significantly damaging the structure of the stent 602. In addition, the stent 602 of such an embodiment may be laser-cut from a single piece of material or may be assembled from a number of different components or manufactured from a various other methods known in the art.

In embodiments, the stent 602 can includes an internal area in which the leaflets 606 can be secured. The leaflets 606 can be formed from a variety of materials, such as autologous tissue, xenograph material, or synthetics as are known in the art. In some embodiments, the leaflets 606 may be provided as a homogenous, biological valve structure, such as porcine, bovine, or equine valves. In some embodiments, the leaflets 606 can be provided independent of one another and subsequently assembled to the support structure of the stent 602. In some embodiments, the stent 602 and the leaflets 606 can be fabricated at the same time, such as may be accomplished using high-strength nano-manufactured NiTi films produced at Advanced Bioprosthetic Surfaces (ABPS), for example. The stent 602 can be configured to accommodate at least two (typically three) of the leaflets 606 but can incorporate more or fewer than three of the leaflets 606.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a medical device.

## Claims

1. A system (100) for delivering an implantable medical device (150) to an implant location, the system comprising:
a control handle portion (106);
a catheter portion (102) coupled to the control handle portion at a proximal end of the catheter portion, the catheter portion comprising an outer shaft (108), wherein the outer shaft comprises:
a plurality of segments (160) arranged in an axial direction (CLA) to form the outer shaft of the catheter portion, each of the plurality of segments being configured to move relative to one another;
a distal portion (104) coupled to a distal end of the outer shaft, the distal portion being configured to receive the implantable medical device; **characterised in that**:
- each of the plurality of segments comprises a body portion having a proximal end and a distal end and forming an inner cavity between the proximal end and the distal end, wherein the inner cavity of one of the plurality of segments is configured to receive the proximal end of an adjacent one of the plurality of segments; and
- the system further comprises
an inner shaft (110) extending through an interior of the plurality of segments from the control handle portion to the distal portion.

2. The system of claim 1, wherein each of the plurality of segments comprises:
a ball portion (210) having a spherical shape; and
a socket portion (212) coupled to the ball portion by a neck portion (306A), wherein the ball portion of one of the plurality of segments is configured to engage with a socket portion of an adjacent one of the plurality of segments.

3. The system of claim 2, wherein the neck portion and the ball portion define an inner channel, and wherein, when the plurality of segments are mated, the inner channel of each of the plurality of segments forms an axial lumen for receiving the inner shaft.

4. The system of claim 3, wherein a proximal opening of the inner channel formed in the ball portion has a larger cross-sectional area relative to a distal opening formed in the neck portion.

5. The system of claim 2, wherein the socket portion (402) comprises:
a first leg (408) extending distally from the neck portion (406); and
a second leg (410) extending distally from the neck portion, wherein:
the first leg and the second leg are formed on opposing sides of each of the plurality of segments,
the first leg and the second leg curve inwards, and
the first leg and the second leg moveably secure the ball portion of one of the plurality of segments when engaged with the socket portion of an adjacent one of the plurality of segments.

6. The system of claim 5, wherein the first leg comprises a slanted distal end (490) and the second leg comprises a slanted distal end and (492) wherein the slanted distal end of the first leg and the slanted distal end of the second leg operate as a stop when one of the plurality of segments moves relative to an adjacent one of the plurality of segments.

7. The system of claim 1, wherein the body portion comprises a lip portion formed at the distal end and wherein an outer surface of the body portion tapers from the lip portion to the proximal end.

8. The system of claim 7, wherein the inner cavity has a frustoconical shape and the lip portion has a circular cross-section.

9. The system of claim 1, wherein the catheter portion further comprises:
a plurality of washers (504) coupled to the inner shaft (503), wherein a washer from the plurality of washers is coupled between adjacent segments of the plurality of segments.

10. The system of any preceding claim,
wherein the plurality of segments is arranged in an axial direction from a control handle portion of the delivery system to a distal portion of the delivery system.

## Patentansprüche

1. System (100) zum Zuführen einer implantierbaren medizinischen Vorrichtung (150) an einen Implantationsort, wobei das System umfasst:
einen Steuergriffabschnitt (106);
einen Katheterabschnitt (102), der mit dem Steuergriffabschnitt an einem proximalen Ende des Katheterabschnitts gekoppelt ist, wobei der Katheterabschnitt einen äußeren Schaft (108) umfasst, wobei der äußere Schaft umfasst:
eine Vielzahl von Segmenten (160), die in einer axialen Richtung (CLA) angeordnet sind, um den äußeren Schaft des Katheterabschnitts zu bilden, wobei jedes aus der Vielzahl von Segmenten konfiguriert ist, um sich relativ zueinander zu bewegen;
einen distalen Abschnitt (104), der mit einem distalen Ende des äußeren Schafts gekoppelt ist, wobei der distale Abschnitt konfiguriert ist, um die implantierbare medizinische Vorrichtung aufzunehmen;
**dadurch gekennzeichnet, dass:**
- jedes aus der Vielzahl von Segmenten einen Körperabschnitt umfasst, der ein proximales Ende und ein distales Ende aufweist und einen inneren Hohlraum zwischen dem proximalen Ende und dem distalen Ende bildet, wobei der innere Hohlraum eines aus der Vielzahl von Segmenten konfiguriert ist, um das proximale Ende eines benachbarten aus der Vielzahl von Segmenten aufzunehmen; und
- das System ferner einen inneren Schaft (110) umfasst, der sich durch ein Inneres der Vielzahl von Segmenten von dem Steuergriffabschnitt zu dem distalen Abschnitt erstreckt.

2. System nach Anspruch 1, wobei jedes aus der Vielzahl von Segmenten umfasst:
einen Kugelabschnitt (210), der eine sphärische Form aufweist; und
einen Pfannenabschnitt (212), der mit dem Kugelabschnitt durch einen Halsabschnitt (306A) gekoppelt ist, wobei der Kugelabschnitt eines aus der Vielzahl von Segmenten konfiguriert ist, um mit einem Pfannenabschnitt eines benachbarten aus der Vielzahl von Segmenten in Eingriff zu kommen.

3. System nach Anspruch 2, wobei der Halsabschnitt und der Kugelabschnitt einen inneren Kanal definieren, und wobei, wenn die Vielzahl von Segmenten zusammengefügt sind, der innere Kanal jedes aus der Vielzahl von Segmenten ein axiales Lumen zum Aufnehmen des inneren Schafts bildet.

4. System nach Anspruch 3, wobei eine proximale Öffnung des in dem Kugelabschnitt gebildeten inneren Kanals eine größere Querschnittsfläche relativ zu einer in dem Halsabschnitt gebildeten distalen Öffnung aufweist.

5. System nach Anspruch 2, wobei der Pfannenabschnitt (402) umfasst:
einen ersten Schenkel (408), der sich distal von dem Halsabschnitt (406) erstreckt; und
einen zweiten Schenkel (410), der sich distal von dem Halsabschnitt erstreckt, wobei:
der erste Schenkel und der zweite Schenkel auf gegenüberliegenden Seiten jedes aus der Vielzahl von Segmenten gebildet sind,
der erste Schenkel und der zweite Schenkel nach innen gekrümmt sind, und
der erste Schenkel und der zweite Schenkel den Kugelabschnitt eines aus der Vielzahl von Segmenten beweglich sichern, wenn sie mit dem Pfannenabschnitt eines benachbarten aus der Vielzahl von Segmenten in Eingriff stehen.

6. System nach Anspruch 5, wobei der erste Schenkel ein abgeschrägtes distales Ende (490) umfasst und der zweite Schenkel ein abgeschrägtes distales Ende (492) umfasst, und wobei das abgeschrägte distale Ende des ersten Schenkels und das abgeschrägte distale Ende des zweiten Schenkels als Anschlag wirken, wenn sich eines aus der Vielzahl von Segmenten relativ zu einem benachbarten aus der Vielzahl von Segmenten bewegt.

7. System nach Anspruch 1, wobei der Körperabschnitt einen an dem distalen Ende gebildeten Lippenabschnitt umfasst und wobei sich eine Außenoberfläche des Körperabschnitts von dem Lippenabschnitt zu dem proximalen Ende verjüngt.

8. System nach Anspruch 7, wobei der innere Hohlraum eine kegelstumpfförmige Form aufweist und der Lippenabschnitt einen kreisförmigen Querschnitt aufweist.

9. System nach Anspruch 1, wobei der Katheterabschnitt ferner umfasst:
eine Vielzahl von Distanzscheiben (504), die mit dem inneren Schaft (503) gekoppelt sind, wobei eine Distanzscheibe aus der Vielzahl von Distanzscheiben zwischen benachbarte Segmente aus der Vielzahl von Segmenten gekoppelt ist.

10. System nach einem der vorstehenden Ansprüche,
wobei die Vielzahl von Segmenten in einer axialen Richtung von einem Steuergriffabschnitt des Zuführsystems zu einem distalen Abschnitt des Zuführsystems angeordnet ist.

## Revendications

1. Système (100) destiné à administrer un dispositif médical implantable (150) à un site d'implantation, le système comprenant :
une partie de poignée de commande (106) ;
une partie de cathéter (102) accouplée à la partie de poignée de commande à une extrémité proximale de la partie de cathéter, la partie de cathéter comprenant une tige externe (108), dans laquelle la tige externe comprend :
une pluralité de segments (160) disposés dans une direction axiale (CLA) pour former la tige externe de la partie de cathéter, chacun de la pluralité de segments étant conçu pour se déplacer les uns par rapport aux autres ;
une partie distale (104) accouplée à une extrémité distale de la tige externe, la partie distale étant conçue pour recevoir le dispositif médical implantable ; **caractérisé en ce que :**
- chacun de la pluralité de segments comprend une partie de corps ayant une extrémité proximale et une extrémité distale et formant une cavité interne entre l'extrémité proximale et l'extrémité distale, dans lequel la cavité interne de l'un de la pluralité de segments est conçue pour recevoir l'extrémité proximale d'un segment adjacent de la pluralité de segments ; et
- le système comprend en outre une tige interne (110) s'étendant à travers un intérieur de la pluralité de segments depuis la partie de poignée de commande jusqu'à la partie distale.

2. Système selon la revendication 1, dans lequel chacun de la pluralité de segments comprend :
une partie de bille (210) ayant une forme sphérique ; et
une partie de douille (212) couplée à la partie de rotule par une partie de col (306A), dans lequel la partie de rotule de l'un de la pluralité de segments est conçue pour venir en prise avec une partie de douille d'un segment adjacent de la pluralité de segments.

3. Système selon la revendication 2, dans lequel la partie de col et la partie sphérique définissent un canal interne, et dans lequel, lorsque la pluralité de segments sont accouplés, le canal interne de chacun de la pluralité de segments forme une lumière axiale pour recevoir la tige interne.

4. Système selon la revendication 3, dans lequel une ouverture proximale du canal interne formée dans la partie sphérique présente une surface de section transversale plus grande par rapport à une ouverture distale formée dans la partie de col.

5. Système selon la revendication 2, dans lequel la partie de douille (402) comprend :
une première patte (408) s'étendant de manière distale à partir de la partie col (406) ; et
une seconde patte (410) s'étendant distalement à partir de la partie col, dans lequel :
la première patte et la seconde patte sont formées sur des côtés opposés de chacun de la pluralité de segments,
la première branche et la seconde branche s'incurvent vers l'intérieur, et
la première patte et la seconde patte fixent de manière mobile la partie rotule de l'un de la pluralité de segments lors de la mise en prise avec la partie cavité d'un segment adjacent de la pluralité de segments.

6. Système selon la revendication 5, dans lequel la première branche comprend une extrémité distale inclinée (490) et la seconde branche comprend une extrémité distale inclinée (492) et dans lequel l'extrémité distale inclinée de la première branche et l'extrémité distale inclinée de la seconde branche servent de butée lorsque l'un de la pluralité de segments se déplace par rapport à un segment adjacent de la pluralité de segments.

7. Système selon la revendication 1, dans lequel la partie de corps comprend une partie de lèvre formée à l'extrémité distale et dans lequel une surface externe de la partie de corps s'effile de la partie de lèvre à l'extrémité proximale.

8. Système selon la revendication 7, dans lequel la cavité interne a une forme tronconique et la partie de lèvre a une section transversale circulaire.

9. Système selon la revendication 1, dans lequel la partie de cathéter comprend en outre :
une pluralité de rondelles (504) accouplées à l'arbre interne (503), dans lequel une rondelle de la pluralité de rondelles est accouplée entre des segments adjacents de la pluralité de segments.

10. Système selon l'une quelconque revendication précédente,
dans lequel la pluralité de segments est agencée dans une direction axiale depuis une partie de poignée de commande du système de pose jusqu'à une partie distale du système de pose.
